# EUROPEAN PATENT SPECIFICATION

(11) **EP 2 246 041 B1**
(45) Date of publication and mention of the grant of the patent: **22.02.2017**
(21) Application number: 09158867.3
(22) Date of filing: 27.04.2009
(51) Int. Cl.: A61K 8/81, A61Q 9/02

(54) **Shaving regimen**
Rasierkur
Régime de rasage

(43) Date of publication of application: 03.11.2010
(73) Proprietor: The Gillette Company LLC, Boston, MA 02127 (US)
(72) Inventor: Stephens, Alison Fiona, Maidenhead, Berkshire SL6 9LA (GB); Cowley, Kevin David, Reading, Berkshire RG2 0QE (GB); Weston, Nigel, Bracknell, Berkshire RG12 0TJ (GB)
(74) Representative: Kohol, Sonia

(56) References cited:
- WO-A-93/18740
- GB-A- 2 236 760
- US-A- 3 072 536
- US-A- 4 585 650
- US-A1- 2003 124 083
- "Clear Shaving Gel" COSMETIC AND TOILETRY, [Online] 17 June 2008 (2008-06-17), XP002547877 Retrieved from the Internet: URL:http://www.cosmeticsandtoiletries.com/ formulating/category/menscare/20104089.htm l> [retrieved on 2009-09-29]

## Description

### FIELD OF THE INVENTION

The present invention concerns the activity of shaving using a razor.

### BACKGROUND OF THE INVENTION

Shaving using a razor is an activity performed regularly by men and women throughout the world. Disadvantages of removing hair in this way are known and reported in the literature and vary from mild redness and irritation through to severe cutting of the skin. There is an ongoing need to provide consumers with a safer and more pleasant shaving experience, which mitigates or avoids these problems.

EP 0 856 308 teaches to use a hydrogel to hydrate the surface of the skin reduce irritation. It also teaches a shaving regimen in which the hydrogel is applied to the skin before applying shaving mousse and then shaving. The hydrogel formulations disclosed in '308 comprise hydrophobic materials, such as fatty acids, to render them as a cream. US3072536 describes the application of a pre shaving lotion comprising homopolymers of acrylamide and acrylic acid and copolymers of said compounds with other suitable monomers to provide lubricity to the skin.

### SUMMARY OF THE INVENTION

According to a first aspect of the invention, a method for shaving an area of skin to remove hair therefrom is provided, the method comprising the step of applying a non-foaming, hydrating composition to the area of skin to be shaved, the non-foaming, hydrating composition comprising:
(i) at least 90% water by weight of the non-foaming, hydrating composition;
(ii) sufficient thickener to give the non-foaming, hydrating composition a viscosity of at least 2000cps, and at most 200,000 cps measured using a Brookfield DVII Viscometer using a T-A spindle at 2.5rpm at 25°C, to form a hydrogel based on polyacrylic acid.
(iii) less than 1%wt, preferably less than 0.5% hydrophobic components, by weight of the non-foaming, hydrating composition;
(iv) less than 1%, preferably less than 0.5% surfactant, by weight of the non-foaming, hydrating composition;
then shaving the area of skin to be shaved using a razor, further comprising the additional steps of waiting for at least one minute after the application of the non-foaming hydrating composition before applying a foam shave preparation to the area of the skin to be shaved after application of the non foaming hydrating composition or before shaving the area of skin.

According to a second aspect of the invention, a kit is provided comprising
(a) a non-foaming, hydrating composition comprising:
   (i) at least 90% water by weight of the non-foaming, hydrating composition;
   (ii) sufficient thickener to give the non-foaming, hydrating composition a viscosity of at least 2000cps, and at most 200,000 cps measured using a Brookfield DVII Viscometer using a T-A spindle at 2.5rpm at 25°C, to form a hydrogel based upon polyacrylic acid.
   (iii) less than 1%wt, preferably less than 0.5% hydrophobic components, by weight of the non-foaming, hydrating composition;
   (iv) less than 1%, preferably less than 0.5% surfactant, by weight of the non-foaming, hydrating composition; and
(b) a foaming shave preparation.

### DETAILED DESCRIPTION OF THE INVENTION

It has been established that the force needed to effect cutting of a hair reduces with the degree of hydration of the hair. A simple way to hydrate hair is to apply water to it, but water has a low viscosity and is repelled by the hydrophobic surface of the skin and hair, so, unless one is in the shower or bath, it is difficult to keep the hair in contact with water for long enough for it to hydrate to a sufficient degree to cause a useful reduction in the cutting force. In order to overcome this problem, it is possible to thicken the water to increase its viscosity and prevent it from running off the skin and hair. One way to create such a viscous, aqueous composition is to mix a carbomer into water to create a hydrogel. EP 0 856 308 teaches to use a hydrogel to hydrate the skin before application of a shaving mousse, but does not discuss either hydration of hair or the concomitant reduction in the cutting force achieved by hydrating the hair.

The present inventors have established that the presence of hydrophobic components within the viscous, aqueous composition may militate against the hydration effects of the water and tend to increase the force needed to cut a hair to which the composition has been applied. Without wishing to be bound by theory, it is believed that the hydrophobic components may preferentially associate with the hydrophobic sebum on the surface of the hair and make it more difficult for the razor blades to gain a purchase. In other words, the blades may have an increased tendency to glide over the hair and cut further down the hair stem from the root or not at all. Ideally, therefore, the viscous, aqueous composition used in the method of the present invention will comprise no hydrophobic components. In practice, consumers often like products to be applied to their skin to have a pleasant smell and, since fragrance oils are generally hydrophobic, a small amount of hydrophobic component is necessary for perfumed compositions.

As used herein, the term "hydrophobic components" includes oils, including hydrocarbon oils, and silicone oils; fats; fatty alcohols, acids and esters; petrolatum and waxes.

A beneficial side-effect of the fact that the compositions used in the method according to the invention contain little or no hydrophobic components is that the composition may additionally comprise little to no surfactant to emulsify the hydrophobic components. Surfactants are skin irritants and so their exclusion assists in reducing skin irritation. In addition, surfactants bind water so may tend to reduce the amount of water available for hydration of the hair. In any case, the present compositions comprise insufficient surfactant for them to foam in use.

Compositions used in the method of the invention comprise as much water as possible in order to achieve the objective of hydrating the skin. Typically, the compositions comprise at least 80%, preferably at least 90% and more preferably at least 95% water by weight of the non-foaming, hydrating composition.

Compositions used in the method of the invention may be thickened in any appropriate way to provide them with a viscosity in the range from 2000 to 200,000 cps, preferably 10,000 to 100,000 cps measured using a Brookfield DVII Viscometer using a T-A spindle at 2.5rpm at 25°C. One appropriate way to thicken such compositions is to thicken them to form a hydrogel. Preferably, the hydrogel is based on polyacrylic acid. Preferred hydrogels are thickened using Carbopol or a Pemulen. The thickener concentration may vary from 1 to 5% by weight of the non-foaming, hydrating composition, preferably from 0.1 to 2% of the non-foaming, hydrating composition. If a carbomer, such as Carbopol, is used, then it is advantageously associated with a base, such as triethanolamine.

Advantageously, compositions used in the method of the invention comprise a humectant. Suitable humectants include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

It can also be advantageous to add a lubricating polymer to compositions used in the method of the invention. As used herein, a "lubricating polymer" is a linear polymer having a molecular weight from 100,000 to 7,000,000 which swells when added to water. Non-limiting examples of such polymers are polyoxyethylene, such as POLYOX, and polyoxypropylene.

Compositions used in the method according to the invention may comprise a variety of additional optional water-soluble ingredients. Non-limiting examples of these additional ingredients include additional water soluble skin care actives such as peptides, vitamins and derivatives thereof such ascorbic acid, vitamin A, vitamin B₃ (e.g., niacinamide) and vitamin B₅ (e.g., panthenol); antioxidants; skin soothing and healing agents such as aloe vera extract, allantoin and the like; chelators and sequestrants.

According to the method of the invention, a person may shave their face using just the non-foaming, hydrating composition and without the addition of any water, other than the water comprised within the non-foaming, hydrating composition. This method has the benefit of being environmentally friendly, since shaving may be performed using only the water within the composition itself and since the composition comprises low levels of surfactant. In such a case, since these compositions do not foam, a user may have difficulty tracking the progress of the shaving, that is establishing which area of skin has already been shaved and which has not (hereinafter referred to as "tracking"). In such a case, an opacifier or colorant may be added to the non-foaming, hydrating composition to facilitate tracking. Any water dispersible or water soluble colorant may be used, such as an organic dye, an encapsulated organic dye or a metal oxide pigment.

For the event that a user decides not to shave with a non-foaming, hydrating composition alone, then a foaming shave preparation may additionally be applied after application of the non-foaming, hydrating composition and before shaving. The use of two such compositions may allow the provision of benefits which cannot be achieved using a single composition alone. For example, while it is possible to provide a foaming shave preparation which comprises a large amount of water, even comparable with the amount of water in non-foaming, hydrating compositions used in the method of the invention, such compositions are generally not able to hydrate hair as well as the defined non-foaming, hydrating compositions. Without wishing to be bound by theory, this may be because the nature of the foam results in far less water being in direct contact with the hair than is the case for a non-foaming, hydrating composition used in the method of the invention. In addition, the large quantities of surfactants present in such compositions may bind a significant proportion of the water such that it is not available to hydrate the hair.

When mixed with foaming shave preparations, the non-foaming, hydrating compositions used in the method of the invention give rise to a rich, creamy mixture. It is known that the presence of hydrophobic components, such as oil, may reduce the stability of a foam. Low oil or oil-free non-foaming, hydrating compositions as defined herein tend not to collapse the subsequently applied foam. This facilitates better tracking.

Advantageously, the user will wait for at least one minute, preferably at least two minutes and more preferably at least three minutes after application of the non-foaming, hydrating composition, before applying the foaming shave preparation. This may to allow an effective hydration of the hair to be removed.

According to a further aspect of the invention a kit is provided comprising
(a) a non-foaming, hydrating composition comprising:
   (i) at least 80%, preferably at least 90% water by weight of the non-foaming, hydrating composition;
   (ii) sufficient thickener to give the non-foaming, hydrating composition a viscosity of at least 2000cps, and at most 200,000 cps measured using a Brookfield DVII Viscometer using a T-A spindle at 2.5rpm at 25°C;
   (iii) less than 1%wt, preferably less than 0.5% hydrophobic components, by weight of the non-foaming, hydrating composition;
   (iv) less than 1%, preferably less than 0.5% surfactant, by weight of the non-foaming, hydrating composition;
(b) a foaming shave preparation.

Advantageously, the kit will be provided with usage instructions to apply the non-foaming, hydrating composition first, optionally to wait at least one minute, preferably at least two minutes, more preferably at least three minutes, then to apply the foaming shave preparation, then to shave. Application of the kit in this way results in a better shave that the application of a foaming shave preparation on its own, because of the hydrating effect of the hydrogel on the hair to be shaved and the resultant reduction in the cutting force required.

The use of the elements of the kit in the defined way may provide a better shave than the use of a foaming shave preparation alone, because it achieves better hydration of the hair to be cut, which, in turn, requires a lower cutting force, in use. This translates into a better shave with reduced skin irritation.

### Example

| **Material** | **%wt** |
|---|---|
| | |
| Deionised water | Qs |
| Carbopol ETD 2020¹ | 0.500 |
| DMDM Hydantoin and butyl carbamate | 0.400 |
| Glycerine | 1.000 |
| Panthenol | 0.500 |
| Disodium EDTA | 0.250 |
| Perfume | 0.150 |
| Triethanolamine | 0.680 |

| | |
|---|---|
| ¹Supplied by Noveon | |

This formulation is made in the following way:
Heat the water and glycerine while stirring (at about 200rpm) to 55°C. Then add disodium EDTA and continue stirring at 55°C until it is fully dissolved. Then add and carefully disperse the Carbopol while stirring (at about 250rpm). Remove from the heat and add the triethanolamine and continue to stir at 200rpm. Then add the panthenol while continuing to stir at 200rpm. When the temperature reaches 45°C, add the DMD Hydantoin/butyl carbamate and continue to stir for 5 minutes. Lastly, add the perfume and continue stirring for about 5 minutes, followed by 1 minute of high shear (at about 7500rpm).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for shaving an area of skin to remove hair therefrom, comprising the step of applying a non-foaming, hydrating composition to the area of skin to be shaved, the non-foaming, hydrating composition comprising:
(i) at least 90% water by weight of the non-foaming, hydrating composition;
(ii) sufficient thickener to give the non-foaming, hydrating composition a viscosity of at least 2000cps, and at most 200,000 cps measured using a Brookfield DVII Viscometer using a T-A spindle at 2.5rpm at 25°C., to form a hydrogel based on polyacrylic acid
(iii) less than 1%wt, preferably less than 0.5% hydrophobic components, by weight of the non-foaming, hydrating composition;
(iv) less than 1%, preferably less than 0.5% surfactant, by weight of the non-foaming, hydrating composition;
then shaving the area of skin to be shaved using a razor, further comprising the additional steps of waiting for at least one minute, after application of the non-foaming hydrating composition before applying a foaming shave preparation to the area of skin to be shaved after application of the non-foaming hydrating composition and before shaving the area of skin.

2. The method of claim 1, comprising the step of waiting for at least two minutes, more preferably at least three minutes after application of the non-foaming, hydrating composition, before applying the foaming shave preparation.

3. The method of any preceding claim, wherein the non-foaming, hydrating composition is free of hydrophobic components.

4. The method of any preceding claim, wherein the non-foaming, hydrating composition is surfactant-free.

5. The method of any preceding claim, wherein the non-foaming, hydrating composition additionally comprises a colorant for tracking purposes.

6. A kit comprising
(a) a non-foaming, hydrating composition comprising:
(i) at least 90% water by weight of the non-foaming, hydrating composition;
(ii) sufficient thickener to give the non-foaming, hydrating composition a viscosity of at least 2000cps, and at most 200,000 cps measured using a Brookfield DVII Viscometer using a T-A spindle at 2.5rpm at 25°C, to form a hydrogel based upon polyacrylic acid.
(iii) less than 1%wt, preferably less than 0.5% hydrophobic components, by weight of the non-foaming, hydrating composition;
(iv) less than 1%, preferably less than 0.5% surfactant, by weight of the non-foaming, hydrating composition and
(b) a foaming shave preparation.

## Patentansprüche

1. Verfahren zum Rasieren einer Hautfläche, um Haar davon zu entfernen, umfassend den Schritt des Auftragens einer nicht schäumenden, hydratisierenden Zusammensetzung auf die zu rasierende Hautfläche, wobei die nicht schäumende, hydratisierende Zusammensetzung Folgendes umfasst:
(i) zu mindestens 90 Gew.-% der nicht schäumenden, hydratisierenden Zusammensetzung Wasser;
(ii) genügend Verdickungsmittel, um der nicht schäumenden, hydratisierenden Zusammensetzung eine Viskosität von mindestens 2 Pa.s (2.000 cP) und höchstens 200 Pa.s (200.000 cP), gemessen mit einem Viskosimeter Brookfield DVII unter Verwendung einer T-A-Spindel mit 2,5 U/min bei 25 °C, zu verleihen, um ein Hydrogel auf Polyacrylsäurebasis zu bilden
(iii) zu weniger als 1 Gew.-%, vorzugsweise weniger als 0,5 Gew.-% der nicht schäumenden, hydratisierenden Zusammensetzung hydrophobe Bestandteile;
(iv) zu weniger als 1 Gew.-%, vorzugsweise weniger als 0,5 Gew.-% der nicht schäumenden, hydratisierenden Zusammensetzung Tensid;
anschließend Rasieren der zu rasierenden Hautfläche mit einem Rasierer, ferner umfassend die zusätzlichen Schritte des Wartens für mindestens eine Minute nach dem Auftragen der nicht schäumenden, hydratisierenden Zusammensetzung, bevor eine schäumende Rasierzubereitung nach dem Auftragen der nicht schäumenden, hydratisierenden Zusammensetzung und vor dem Rasieren der Hautfläche auf die zu rasierende Hautfläche aufgetragen wird.

2. Verfahren nach Anspruch 1, umfassend den Schritt des Wartens für mindestens zwei Minuten, mehr bevorzugt mindestens drei Minuten, nach dem Auftragen der nicht schäumenden, hydratisierenden Zusammensetzung, bevor die schäumende Rasierzubereitung aufgetragen wird.

3. Verfahren nach einem der vorstehenden Ansprüche, wobei die nicht schäumende, hydratisierende Zusammensetzung frei von hydrophoben Bestandteilen ist.

4. Verfahren nach einem der vorstehenden Ansprüche, wobei die nicht schäumende, hydratisierende Zusammensetzung tensidfrei ist.

5. Verfahren nach einem der vorstehenden Ansprüche, wobei die nicht schäumende, hydratisierende Zusammensetzung zusätzlich einen Farbstoff für Verfolgungszwecke umfasst.

6. Set, umfassend:
(a) eine nicht schäumende, hydratisierende Zusammensetzung, umfassend:
(i) zu mindestens 90 Gew.-% der nicht schäumenden, hydratisierenden Zusammensetzung Wasser;
(ii) genügend Verdickungsmittel, um der nicht schäumenden, hydratisierenden Zusammensetzung eine Viskosität von mindestens 2 Pa.s (2.000 cP) und höchstens 200 Pa.s (200.000 cP), gemessen mit einem Viskosimeter Brookfield DVII unter Verwendung einer T-A-Spindel mit 2,5 U/min bei 25 °C, zu verleihen, um ein Hydrogel auf Polyacrylsäurebasis zu bilden;
(iii) zu weniger als 1 Gew.-%, vorzugsweise weniger als 0,5 Gew.-% der nicht schäumenden, hydratisierenden Zusammensetzung hydrophobe Bestandteile;
(iv) zu weniger als 1 Gew.-%, vorzugsweise weniger als 0,5 Gew.-% der nicht schäumenden, hydratisierenden Zusammensetzung Tensid und
(b) eine schäumende Rasierzubereitung.

## Revendications

1. Procédé de rasage d'une zone de peau pour en retirer les poils, comprenant l'étape d'application d'une composition hydratante non moussante sur la zone de peau à raser, la composition hydratante non moussante comprenant :
(i) au moins 90 % d'eau en poids de la composition hydratante non moussante ;
(ii) une quantité suffisante d'épaississant pour donner à la composition hydratante non moussante une viscosité d'au moins 2 Pa.s (2000 cP) et d'au plus 200 Pa.s (200 000 cP), mesurée à l'aide d'un viscosimètre Brookfield DVII en utilisant un mobile cylindrique T-A à 2,5 tr/min à 25 °C., pour former un hydrogel à base d'acide polyacrylique
(iii) moins de 1 % en poids, de préférence moins de 0,5 % de composants hydrophobes, en poids de la composition hydratante non moussante ;
(iv) moins de 1 %, de préférence moins de 0,5 % d'agent tensioactif, en poids de la composition hydratante non moussante ;
puis le rasage de la zone de peau à raser en utilisant un rasoir, comprenant en outre les étapes supplémentaires consistant à attendre pendant au moins une minute, après application de la composition hydratante non moussante avant l'application d'une préparation de rasage moussante sur la zone de peau à raser après application de la composition hydratante non moussante et avant le rasage de la zone de peau.

2. Procédé selon la revendication 1, comprenant l'étape consistant à attendre pendant au moins deux minutes, plus préférablement au moins trois minutes après application de la composition hydratante non moussante, avant application de la préparation de rasage moussante.

3. Procédé selon l'une quelconque des revendications précédentes, dans lequel la composition hydratante non moussante est exempte de composants hydrophobes.

4. Procédé selon l'une quelconque des revendications précédentes, dans lequel la composition hydratante non moussante est sans agent tensioactif.

5. Procédé selon l'une quelconque des revendications précédentes, dans lequel la composition hydratante non moussante comprend en outre un colorant à des fins de repérage.

6. Trousse comprenant
(a) une composition hydratante non moussante comprenant :
(i) au moins 90 % d'eau en poids de la composition hydratante non moussante ;
(ii) une quantité suffisante d'épaississant pour donner à la composition hydratante non moussante une viscosité d'au moins 2 Pa.s (2000 cP) et d'au plus 200 Pa.s (200 000 cP), mesurée à l'aide d'un viscosimètre Brookfield DVII en utilisant un mobile cylindrique T-A à 2,5 tr/min à 25 °C., pour former un hydrogel à base d'acide polyacrylique.
(iii) moins de 1 % en poids, de préférence moins de 0,5 % de composants hydrophobes, en poids de la composition hydratante non moussante ;
(iv) moins de 1 %, de préférence moins de 0,5 % d'agent tensioactif, en poids de la composition hydratante non moussante et
(b) une préparation de rasage moussante.
